# EUROPÄISCHE PATENTSCHRIFT

(11) **EP 0 697 923 B1**
(45) Veröffentlichungstag und Bekanntmachung des Hinweises auf die Patenterteilung: **31.01.2001**
(21) Anmeldenummer: 94917620.0
(22) Anmeldetag: 11.05.1994
(51) Int. Cl.: B09B 3/00, A61L 11/00

(54) **ENTSORGUNGSVORRICHTUNG FÜR ABFÄLLE**
WASTE DISPOSAL DEVICE
DISPOSITIF D'ELIMINATION DE DECHETS

(30) Priorität: 11.05.1993 DE 4315735
(43) Veröffentlichungstag der Anmeldung: 28.02.1996
(73) Patentinhaber: Maihofer, Elisabeth, 6986 Novaggio (CH)
(72) Erfinder: MAIHOFER, Wilhelm, Eugen, CH-6986 Novaggio (CH)
(74) Vertreter: Schwabe, Hans-Georg, Dipl.-Ing.
(86) Internationale Anmeldenummer: EP9401523
(87) Internationale Veröffentlichungsnummer: WO9426433

(56) Entgegenhaltungen:
- EP-A- 0 410 306
- WO-A-90/12601
- WO-A-93/04704
- DE-A- 3 911 420

## Beschreibung

Die Erfindung betrifft eine Vorrichtung für die Entsorgung vorzugsweise infektiöser Abfälle sowie ein Fahrzeug, auf dem die erfindungsgemäße Vorrichtung angeordnet werden kann, gemäß den Oberbegriffen der Patentansprüche 1 und 28.

Eine derartige Vorrichtung weist eine Behandlungskammer auf, die mit einem Behandlungsabschnitt mit Stellplätzen für die Abfälle aufnehmenden Behälter versehen ist. Mindestens eine Wärmequelle ist in der Behandlungskammer angeordnet. Verbindungsorgane, vorzugsweise Hohlnadeln, die an Ver- bzw. Entsorgungsleitungen anschließbar sind, sind ebenfalls in der Behandlungskammer vorgesehen.

In Krankenhäusern, Gemeinschaftspraxen, Bio- und Genlabors und dergleichen fallen häufig große Mengen von Abfällen an, die als infektiöser Abfall zu gelten haben. Derartige infektiöse Abfälle müssen als Sondermüll entsorgt werden.

Krankenhauseigene Anlagen sind hinsichtlich der Anschaffungskosten, der Betriebs- und Unterhaltungsaufwendungen etc. kaum rentabel, da derartige Anlagen durch Krankenhäuser der üblichen Größe bzw. durch Gemeinschaftspraxen, Bio- oder Genlabors der üblichen Größe nicht ausgenutzt werden können. Das gleiche gilt für Verbrennungsanlagen.

Ferner wird die Entsorgung derzeit so praktiziert, daß der infektiöse Müll in dichten, die Abfälle aufnehmenden Behältern von den Müllerzeugern zu einer Entsorgungsanlage, z.B. einer Sondermüll-Verbrennungsanlage, transportiert wird. Die durchschnittliche Fahrstrecke beträgt dabei zwischen 280 und 350 km und der zu transportierende Müll kann nicht vor seinem Abtransport gepreßt werden, um einen effektiven Transport und eine effektive Entsorgung gewährleisten zu können. Zudem kann derartigen Mülltransporten auf der Transportstrecke etwas zustoßen, was zu Verseuchungen der Umwelt führen kann. Die den Sondermüll aufnehmenden Transportbehälter müssen verbrannt werden und können nicht als Rohstoff zurückgewonnen werden, was bei ca. 2 kg Kunststoffmaterial pro Transportbehälter zu ganz erheblichen Verlusten führt.

Aus der WO 93/04704 sind ein Verfahren und eine Vorrichtung zum Desinfizieren oder Sterilisieren von infektiösem Abfall, wie Krankenhausmüll, bekannt. Diese Vorrichtung, die auf einem Fahrzeug installiert ist, weist einen Behandlungsabschnitt mit Stellplätzen für die Abfälle aufnehmende Behälter auf. Energiequellen in Form von Mikrowellengeneratoren sind angeordnet, um die für einen Desinfektionsvorgang erforderliche Energie bereitzustellen. Mittels eines sogenannten Funktionskopfes wird der Deckel eines jeweiligen Behälters großflächig durchbrochen und über ein an dem Funktionskopf angeordnetes Zuführrohr wird Mikrowellenstrahlung in den Behälter eingeleitet. Auch eine Zuführleitung zum Zuführen von Wasser in den Behälter ist angeordnet. Nachteilig ist hier, daß die Entsorgung der aus dem Behälter abgesaugten Desinfektionsabgase und die Steuerung und Kontrolle der Bedingungen innerhalb des Behälters mit dem zu desinfizierenden Müll nicht in einer ausreichenden Form möglich ist.

Aus der WO 90/12601 ist ein Verfahren zur Sterilisation von Medizinalabfällen bekannt, bei dem zunächst eine Desinfektionskammer, in der Müll enthaltende Behälter angeordnet sind, zusammen mit den Behältern mit einem inerten Gas unter Reduktion des Sauerstoffgehalts der in der Kammer bzw. dem Behälter befindlichen Atmosphäre, gespült wird. Eine ozonhaltige Lösung wird in die Behälter bzw. die Reaktionskammer eingefüllt und unter Energiezufuhr wird eine Heißdampf-atmosphäre einer ausreichenden Temperatur erzeugt, um die infizierten Abfälle zu desinfizieren. Die hier getroffenen Maßnahmen sind ebenfalls nicht ausreichend, um die vollständige Desinfektion hochinfektiösen Mülls garantieren zu können.

Aus der EP 0 410 306 A2 sind ein Verfahren und eine Vorrichtung zur Sterilisation von Müll, insbesondere Krankenhausmüll, bekannt. Auch hier wird der Inhalt eines Behälters über eine Dampfsterilisation desinfiziert, wobei als Energiequelle Mikrowellengeneratoren eingesetzt werden. Allerdings sind auch hier Mängel bei der Kontrolle der Desinfektionsparameter zu beanstanden. Darüber hinaus ist auch die Verseuchung der Umwelt zu befürchten, da bei dieser Vorrichtung die erforderlichen dichten und sterilen Anschlüsse an die mit dem Krankenhausmüll bzw. desinfizierten Müll beladenen Behälter nicht in der wünschenswerten Weise gewährleistet.

Derlei Mängel treten auch bei den oben genannten und der im folgenden erörterten Vorrichtung aus dem Stand der Technik auf.

Die DE-A-39 11 420 zeigt eine Vorrichtung bzw. ein Verfahren zur Entsorgung von insbesondere kohlenwasserstoffhaltigen Flüssigkeiten, die in Küchen- und Haushaltsgeräten zum Einsatz gelangen. Hier werden die geschlossenen Leitungssysteme von entsprechenden Geräten beispielsweise angebohrt, um die zu entsorgenden Flüssigkeiten zu saugen. Es handelt sich hierbei jedoch nicht um infektiösen Müll, an dessen Entsorgung unter anderem auch von den Gesundheitsämtern ganz besondere Anforderungen gestellt werden.

Es ist die Aufgabe der vorliegenden Erfindung, eine Vorrichtung bzw. ein Fahrzeug zur Entsorgung von infektiösem Abfall vorzuschlagen, die bzw. das den Nachteilen des Standes der Technik soweit als möglich Abhilfe verschafft. Insbesondere ist es die Aufgabe der vorliegenden Erfindung, eine Vorrichtung vorzuschlagen, die gegenüber bekannten Systemen robust, wenig anfällig und zuverlässig ist.

Diese Aufgabe wird durch eine Vorrichtung gemäß dem Patentanspruch 1 bzw. ein Fahrzeug gemäß dem Patentanspruch 28 gelöst.

Vorteilhafte Ausgestaltungen gehen aus den in den Unteransprüchen definierten Merkmalen hervor.

Erfindungsgemäß sind zumindest für einige, vorzugsweise für jeden der Sammelleitungsabschnitte Einsteckhohl- bzw. Injektionsnadeln vorgesehen, die zumindest vertikal bewegbar sind. Ferner ist oberhalb des Verbindungsendes und/oder der Anschlußöffnungen der Einsteckhohl- bzw. Injektionsnadeln eine Dichteinrichtung, beispielsweise eine Silikonscheibe, ein Silikonkissen, eine Gummilatte oder dergleichen, angeordnet, die einen Behälter im eingestochenen Zustand gegenüber der Umgebung bzw. Außenwelt isoliert. Die bei bekannten Vorrichtungen erforderlichen umfangreichen Schlauch- und Kabelgewirre, die zu häufigen Ausfällen, Unsicherheiten und zu Fehlfunktionen bekannter Entsorgungsvorrichtungen führen, erübrigen sich gemäß der Erfindung.

Um gemäß der Erfindung die Dichtigkeit der Behälter während der Sterilisation zu gewährleisten, ist oberhalb des Verbindungsendes und/oder der Anschlußöffnungen des jeweiligen Verbindungsorgans mindestens eine Dichteinrichtung, beispielsweise eine Silikonscheibe, ein Silikonkissen, eine Gummiplatte oder dergleichen, angeordnet, die die Umgebung, in der ein Behälter mit Abfällen perforiert wird, abdichten kann. Um die Dichtigkeit ferner abzusichern, kann die Dichteinrichtung abgestützt werden, indem sie auf der dem abzudichtenden Behälter abgewandten Seite, beispielsweise an einer Metallplatte, anliegt.

Anstelle der Einstech- bzw. Injektionshohlnadeln sind auch mit einem Gewinde bzw. einer Bajonettverbindung versehene Enden eines Verbindungsorgans verwendbar, die mit entsprechend ausgebildeten Öffnungen an den Behältern in Verbindung treten können.

Dabei sind auf den Stellplätzen Behälter, insbesondere abgedichtete, die Abfälle aufnehmende Behälter angeordnet, die von den Verbindungsorgangen punktiert werden, wenn diese zusammen mit den Sammelleitungsabschnitten abgesenkt werden.

Eine unter Umständen gleichwertige Lösungsmöglichkeit kann sich ergeben, wenn die Verbindungsorgane feststehen und die Behälter auf und ab bewegt werden.

Die Sammelleitungsabschnitte bilden vorteilhafterweise eine rahmenartige Anordnung, die mittels einer Hydraulikeinrichtung einer Zahnstange, einem Schneckentrieb oder dergleichen, vorzugsweise in vertikaler Richtung auf bzw. ab bewegt werden kann.

Die gemäß der Erfindung zu verwendende Vorrichtung in Verbindung mit den Behältern ergibt die Möglichkeit, die Behälter bzw. deren Material zurückzugewinnen, was bei einem Umsatz von mehreren Millionen Behältern allein in Deutschland zu ganz erheblichen Einsparungen an Material und zu einer beachtlichen Umweltentlastung führt.

Vorteilhafterweise sind die jeweiligen Sammelleitungsabschnitte, die flüssige und/oder gasförmige Medien zu den die Abfälle aufnehmenden Behältern zu- bzw. ableiten, im wesentlichen mittig an die jeweiligen Ver- bzw. Entsorgungen angeschlossen. Auf diese Weise ist gewährleistet, daß, wenn eine Vielzahl von Behältern mit Abfällen hintereinander angeordnet sind, sämtliche Behälter im wesentlichen mit dem gleichen Leitungswiderstand, beispielsweise mit Wasser befüllt werden können, von Wasserdampf bzw. Gasen entsorgt werden können bzw. mit Druckluft beaufschlagt werden können, so daß sich auch über eine Vielzahl von Behältern mit Abfällen vollkommen gleichmäßige Bedingungen einstellen lassen. Eine zuverlässige und gleichmäßige Sterilisation läßt sich auf diese Art und Weise regelmäßig veranlassen, während bei den bekannten Vorrichtungen, die eine Vielzahl von unterschiedlich langen Ver- bzw. Entsorgungsleitungen aufwiesen, in verschiedenen Behältern mit infektiösem Abfall derart unterschiedliche Infektionsbedingungen auftraten, daß keineswegs gewährleistet war, daß der infektiöse Abfall mit Sicherheit sterilisiert wurde.

Die Unempfindlichkeit der erfindungsgemäßen Vorrichtung läßt sich ferner steigern, wenn sämtliche Sammelleitungsabschnitte in einem jeweiligen oder gemeinsamen Sammelrohr bzw. einer Sammeleinrichtung angeordnet sind, an der vorzugsweise auch die Verbindungsorgane, beispielsweise Injektionsnadeln, abgestützt sind.

Die Verbindungsorgane weisen ein Verbindungsende auf, an dem mehrere Leitungen enden bzw. münden. Dieses Verbindungsende weist vorteilhafterweise eine Schneidfläche auf, um einen jeweiligen Behälter mit Abfall an einer vorzugsweise vorgegebenen Stelle perforieren bzw. punktieren zu können. Die Seitenwände der Verbindungsorgane weisen in vorteilhafter Weise die Anschlußöffnungen der Ver- bzw. Entsorgungsleitungen auf. Dieses hat den enormen Vorteil, daß die der Schneidfläche zugeordnete Öffnung, falls diese eine Öffnung aufweist, durchaus verstopfen kann, ohne daß dadurch der eigentliche Sterilisationsvorgang behindert werden kann. Trifft nämlich das Verbindungsorgan beim Einstechen in einen Behälter mit infektiösen Abfällen beispielsweise auf Wattematerialien oder dergleichen und wird durch diese zugesetzt, so kann der Sterilisationsvorgang nach wie vor durch die seitlichen Austrittsöffnungen ungehindert ablaufen.

Da die den infektiösen Abfall aufnehmenden Behälter in der Praxis meist in einer festen Formation in die Behandlungskammer eingeführt werden, sollten die Sammelleitungsabschnitte und dementsprechend auch die Verbindungsorgane längs oder senkrecht zu der Formation der die infektiösen Abfälle enthaltenden Behälter angeordnet sein.

Anstelle der Verbindungsorgane, insbesondere Injektionsnadeln, können auch die Behälter bzw. sowohl die Behälter als auch die Verbindungsorgane zumindest vertikal verfahrbar sein.

Die in den Sammelleitungsabschnitt aufgenommenen Ver- bzw. Entsorgungsleitungen weisen vorzugsweise Leitungen für die flüssigen und/oder gasförmigen Medien auf, die zum Zwecke einer sicheren Sterilisation zu- bzw. abführbar sein sollten. Insbesondere gehört zu diesen Leitungen eine Vakuumleitung, eine Wasserzuleitung und eine Druckluftzuleitung, wobei die Vakuumleitung auch gleichzeitig für die Entsorgung des Wasserdampfes herangezogen werden kann. Zusätzlich können elektrische Versorgungs- und Steuerleitungen angeordnet sein.

Weisen die Ver- bzw. Entsorgungsleitungen für die flüssigen und/oder gasförmigen Medien einen großen, vorzugsweise größtmöglichen Querschnitt auf, so läßt sich über diese Leitungen ein Druckausgleich zwischen den die infektiösen Abfälle aufnehmenden Behältern gewährleisten. Da die die infektiösen Abfälle aufnehmenden Behälter häufig mit unterschiedlichen Materialien mit unterschiedlichen Wasser- bzw. Flüssigkeitsanteilen und unterschiedlichen Mengen an infektiösem Müll aufweisen, kann auf diese Weise während des Sterilisationsvorganges ein Druck- bzw. Bedingungsausgleich durchgeführt werden, wodurch sich auch bei unterschiedlichem Befüllungsgrad der zu desinfizierenden Behälter in sämtlichen Behältern vollkommen gleichmäßige Desinfektionsbedingungen einstellen lassen, wodurch eine Sterilisation mit größtmöglicher Zuverlässigkeit erzielt werden kann.

Um die Sterilisationsbedingungen zumindest zeitweise überprüfen zu können, ist vorzugsweise an dem in die Behälter mit dem Abfall eingeführten Abschnitt eines jeweiligen Verbindungsorgans, vorzugsweise einer Injektionsnadel, mindestens ein Meßfühler angeordnet. Dieser Meßfühler kann gegebenenfalls eine Temperatur-, eine Druck- und/oder eine Feuchtemessung vornehmen. Dabei kann es sich um ein Widerstandsthermometer, einen druckempfindlichen Kondensator oder dergleichen handeln. Selbstverständlich kann im Verlaufe der Ver- bzw. Entsorgungsleitungen bzw. an den jeweiligen Ver- bzw. Entsorgungsquellen, beispielsweise einer Vakuumpumpe, einem Wassertank, etc. mindestens ein jeweiliger Meßfühler angeordnet sein, der ebenfalls als Temperatur-, Druck- und/oder Feuchtigkeitsmeßeinrichtung ausgebildet sein kann, um eine größtmögliche Kontrolle über sämtliche Parameter des Sterilisationsvorganges gewährleisten zu können.

In der Entsorgungsleitung für den Wasserdampf bzw. in der Vakuumleitung ist vorteilhafterweise eine Filtereinrichtung, z.B. ein Feinstfilter, angeordnet, um gegebenenfalls Bakterien, Viren usw. zurückzuhalten.

Von besonderem Vorteil ist es, wenn vor der Filtereinrichtung eine Kühleinrichtung, vorzugsweise ein Kondensator, angeordnet ist, um den kondensierbaren Flüssigkeitsanteil in dem abzuführenden Wasserdampf so weit als möglich zu kondensieren, da dieser eine gegebenenfalls angeordnete Feinstfiltereinrichtung in kürzester Zeit zusetzen würde. Das von der Kühleinrichtung zurückgewonnene Kondensat kann anschließend zusätzlich sterilisiert werden, um später in einen Sterilisationsflüssigkeitstank, vorzugsweise einen Wassertank, zurückgeleitet zu werden, von dem aus wiederum über Versorgungsleitungen, die in einem Sammelleitungsabschnitt angeordnet sein können, wieder in einen mit Abfall gefüllten Behälter zu einer nachfolgenden Sterilisation eingeführt werden kann. Hierdurch ist es möglich, die in der erfindungsgemäßen Vorrichtung erforderlichenfalls zu bevorratende Flüssigkeitsmenge relativ gering zu halten, so daß ein Fahrzeug, das die erfindungsgemäße Vorrichtung prägt, einen relativ geringen Vorrat an für die Sterilisation erforderlichen Wassers mit sich führen muß, da dieses nach der Sterilisation zu einem größeren Anteil zurückgewonnen werden kann.

Vorteilhafterweise wird die Verdampfung und damit die Sterilisation durch Mikrowellen bewirkt, die durch Mikrowellengeneratoren erzeugt werden, die einem jeweiligen Behälter mit infektiösem Abfall zuordenbar sind.

Vorteilhafterweise werden die Stellplätze für die die Abfälle aufnehmenden Behälter auf einer bewegbaren Einrichtung angeordnet, die vorzugsweise schubladenartig in die Behandlungskammer einführbar ist. Hierdurch ist es möglich, die Bauhöhe der erfindungsgemäßen Vorrichtung niedrig auszuführen, da die Behälter außerhalb der erfindungsgemäßen Vorrichtung aufgeladen werden können und Personen lediglich zu Wartungszwecken in die Vorrichtung selbst gelangen müssen. Durch die geringe Bauhöhe müssen die Behälter bzw. die Verbindungsorgane, die nachfolgend als "Injektionsnadel" bezeichnet werden, relativ dicht über den jeweiligen Stellplätzen der Behälter unmittelbar vor dem Sterilisationsvorgang anzuordnen. Auf diese Weise brauchen nur geringe vertikale Strecken überbrückt zu werden, was die Anfälligkeit der erfindungsgemäßen Vorrichtung und damit deren Zuverlässigkeit abermals erhöht.

Es ist ersichtlich, daß die der Erfindung zugrundeliegende Aufgabe durch eine Kombination der erfindungsgemäßen bzw. der vorteilhaften Ausgestaltungsmerkmale noch vorteilhafter und wirksamer gelöst werden kann. Dieses gilt auch für die im folgenden aufgeführten Merkmale.

Die bewegbare Einrichtung, die vorzugsweise schubladenartig ausgestaltet ist, weist vorteilhafterweise eine Auflageplatte auf. Die Einrichtung und/oder die Auflageplatte sind zumindest teilweise aus einem im wesentlichen metallische Eigenschaften aufweisenden Materialien nicht enthaltenden Material, vorzugsweise Glas, insbesondere Polycarbonat, Teflon und/oder Kunststoff oder dergleichen ausgebildet, damit sich den Mikrowellen der gegebenenfalls unter der Platte angeordneten Mikrowellengeneratoren kein Hindernis entgegenstellt. Eine aus einem leitenden Material bestehende Platte würde zu Reflexionen der Mikrowellen führen, die eine effektive Ausnutzung der Mikrowellenenergie zur Sterilisation stark einschränkten bzw. behindern würden.

In vorteilhafter Weise wird die Platte über mindestens einen Abstandshalter gegenüber dem Boden der Behandlungskammer abgestützt. Über diesen Abstandshalter kann die Platte und damit die bewegbare Einrichtung innerhalb der Behandlungskammer bewegbar sein. Das heißt, die Abstandshalter können auf im Boden oder am Boden der Behandlungskammer angeordneten Rollenleisten abgestützt sein. Andererseits können auch Gewinde- oder Schneckentriebe oder dergleichen mehr am oder im Boden der Behandlungskammer angeordnet sein, die an einem bzw. den Abstandshaltern angreifen und damit die schubladenartige Einrichtung, die nachfolgend als "Sterilisationslade" bezeichnet wird, in eine gewünschte Stellung zu bewegen.

Auf bzw. an der Platte können Vertiefungen und/oder Erhebungen ausgebildet sein, die vorzugsweise eine Positionierung der die Abfälle aufnehmenden Behälter, im folgenden "Abfallbehälter" genannt, zu den Injektionsnadeln und/oder deren Abstützung gegenüber den Injektionsnadeln ermöglichen. Auch die Vertiefungen bzw. Erhebungen sind aus einem für Mikrowellen nicht hinderlichen Material, vorzugsweise einem dielektrischen Material.

An einem Endbereich der Sterilisationslade ist ein Endabschnitt angeordnet, der die Behandlungskammer im wesentlichen verschließt, wenn die Sterilisationslade in der Behandlungskammer in einer Behandlungsstellung ist. Hierdurch kann die Behandlungskammer automatisch geschlossen werden, wenn die Sterilisationslade in die Behandlungskammer eingeführt ist, wobei die Behandlungskammer durch den Endabschnitt insbesondere mikrowellendicht verschließbar ist.

Vorzugsweise weist der Endabschnitt eine Vorspannung auf, die die Sterilisationslade mit der Behandlungskammer in einen Eingriff mit verbesserter Dichtwirkung treten läßt. Diese Vorspannung kann durch Materialeigenschaften und eine Verwinkelung des Endabschnitts zur Wandung der Behandlungskammer oder Federeinrichtungen erzeugt werden.

Um Keimbildungen vorzubeugen und ein etwaiges Entweichen von Mikroben oder Viren noch zuverlässiger auszuschließen, können einige bzw. sämtliche Ver- bzw. Entsorgungsleitungen zumindest bereichsweise mit Heizeinrichtungen versehen sein, die hohe Temperaturen erzeugen und damit Restkeime und dergleichen abtöten. Hier bieten sich insbesondere die Wasserzu- und Kondensatableitungen an, die mit elektrischen Widerstandsheizungen umwickelt werden können.

Um das Entweichen von unangenehmen Gerüchen aus der erfindungsgemäßen Vorrichtung auszuschließen, können in der Abluftleitung, beispielsweise der Ableitung für die Vakuumpumpe, die auch zur Dampfentsorgung dienen kann, Filtereinrichtungen, beispielsweise ein Aktivkohlefilter oder dergleichen, angeordnet sein.

Ganz besondere Vorteile ergeben sich, wenn die erfindungsgemäße Vorrichtung auf einem Fahrzeug, beispielsweise einem Lastkraftwagen, angeordnet wird.

Dabei kann im Kopfraum der Behandlungskammer bzw. oberhalb der Behandlungskammer im Fahrzeug ein Wasservorratstank angeordnet sein, der insbesondere mindestens einen Vertiefungsbereich aufweist, der an die Injektionsnadeln anschließbar ist.

Die Anordnung der erfindungsgemäßen Vorrichtung auf einem Fahrzeug ist besonders vorteilhaft, da am Herstellungsort der infektiösen Abfälle in entsprechend vorbereiteten Behältern eine Desinfektion vorgenommen werden kann. Diese Desinfektion kann auch während der Fahrt zu einer Müllpresse und zu einer Hausmüllentsorgungsanlage vorgenommen werden. Weite Entfernungen sind auf diese Weise nicht zurückzulegen, da der Sondermüll unmittelbar vor oder während des Transportes zu Hausmüll umgewandelt wird.

Eine Prozessor- bzw. Computersteuerung kann in die Vorrichtung bzw. in das erfindungsgemäße Fahrzeug aufgenommen werden, um eine größtmögliche Sicherheit unabhängig von der Aufmerksamkeit eines Bedienungspersonals zu gewährleisten. Diese entsprechende Steuervorrichtung sorgt für gleichmäßige Desinfektionsvorgänge und kann im Störungsfall dafür sorgen, daß das Bedienungspersonal auf Fehlfunktionen aufmerksam gemacht wird, beispielsweise durch Warnleuchten und eine Verriegelung der Behandlungskammer, um die Entsorgung von infektiösem Abfall als Hausmüll zu verhindern.

Die Desinfektionslade kann sowohl von hinten als auch seitlich in die auf dem Fahrzeug installierte Behandlungskammer eingeführt werden.

Zusammenfassend können folgende Vorteile der vorliegenden Erfindung festgehalten werden:
Hochinfektiöse Abfälle müssen nicht mehr über große Entfernungen transportiert werden, um als Sondermüll entsorgt werden zu können. Die Kontermination nicht infizierter Bereiche und das Verschleppen von Keimen wird ausgeschlossen. Die Desinfektion direkt in Einweg-Recycling-Behälter, ohne chemische Mittel, ohne Geruchsbelästigung und ohne Umweltbelastung wird möglich. Eine Volumenreduzierung von ca. 80% durch Containerpresse und eine problemlose Entsorgung wird möglich. Zusätzlich ist es möglich, Müll zu reduzieren, da Speise- und Küchenabfälle in der gleichen Anlage, jedoch in entsprechend gekennzeichneten Behältern desinfiziert und beispielsweise der Tiermast zugeführt werden können. Dank besonders günstiger Entsorgung können personalintensive Sortiervorgänge erübrigt werden und die Gefahr von Fehlzuordnungen entfällt. Die Müllverursacher können ohne größere Investitionen eine kostengünstige Entsorgung sicherstellen, wobei aufgrund der Kostengünstigkeit eine Entsorgung "unter der Hand" nicht mehr zu befürchten ist, da derartige, möglicherweise gegen Vorschriften verstoßende Entsorgungen nicht mehr kostengünstiger sind als die durch die erfindungsgemäße Vorrichtung zu bewerkstelligende Entsorgung.

Ein beispielhafter Funktionsablauf mit der erfindungsgemäßen Vorrichtung kann wie folgt beschrieben werden:

Nach dem Befüllen/Verschließen der Behälter werden diese, beispielsweise 10 Stück, auf der Desinfektionslade der erfindungsgemäßen Vorrichtung abgestellt. Nach dem Drücken der Einlauftaste läuft ein in eine Steuervorrichtung eingespeichertes Programm vollautomatisch ab. Zunächst werden die beispielsweise 10 Abfallbehälter in die Behandlungskammer eingezogen. Die Injektionsnadeln, beispielsweise 10 Injektionsnadeln, werden zusammen mit den Sammelleitungsabschnitten abgesenkt und mit gleichzeitiger Abdichtung der Einstichstelle an den Behältern abgesenkt. Aus den Behältern wird die Luft über zwischengeschaltete Bakterienfilter, beispielsweise Feinstfilter, abgezogen. In jeden der Behälter wird ca. 1 Liter Wasser über die Injektionsnadeln eingespritzt. Anschließend werden eine entsprechende Anzahl von Mikrowellen-Generatoren mit beispielsweise jeweils 1,2 Kilowatt Leistung eingeschaltet, bis ein Sattdampfaufbau auf mindestens ca. +105°C bei einem leichten Überdruck von beispielsweise 0,2 Bar erreicht ist.

Anschließend läuft die Desinfektion unter Berücksichtigung aller behördlichen Vorschriften und Auflagen des Bundesgesundheitsamtes ab. Die Abtötung des geforderten Wirkungsspektrums A, B und C wird je nach Sondermüllkategorie durchgeführt. Gegebenenfalls kann über die Steuervorrichtung ein Protokollausdruck über die Chargennummer einer bestimmten Abfallcharge von Behältern zugeordnet werden, um einen Nachweis für die gesetzmäßige Entsorgung führen zu können, wobei Datum, Zeit, Temperatur und Druck sowie gegebenenfalls die Art des infektiösen Sondermülls, der in Hausmüll umgewandelt wird, aufgeführt werden.

Die anschließende Entlüftung der desinfizierten Abfallbehälter läuft ebenfalls durch die Injektionsnadeln bzw. Hohlnadeln ab, wobei der abgezogene Dampf kondensiert und das Kondensat für die weitere Verwendung rückgewonnen werden kann. Anschließend können die Behälter aus der erfindungsgemäßen Vorrichtung entfernt werden und der desinfizierte Müll mit oder ohne den Behälter zur Volumenreduzierung verpreßt werden. Einer anschließenden Entsorgung mit dem üblichen Hausmüll steht dann nichts mehr entgegen.

Die Heizleistung wird in der Regel durch vorzugsweise wassergekühlte Mikrowellen-Generatoren mit einer üblichen Hochfrequenz von 2,45 GHz und einer Leistung von beispielsweise 1,2 Kilowatt erzeugt. Derartige Generatoren können innerhalb weniger Minuten die benötigte Leistung für die Desinfektion bereitstellen.

Es ist ferner festzuhalten, daß die erfindungsgemäße Vorrichtung/Fahrzeug auch zur Desinfektion und sogar zur Aufbereitung von Wasser, beispielsweise in Katastrophengebieten, etwa nach Erdbeben, verwendbar ist. So kann Wasser in den Behältern sterilisiert oder auch verdampft werden, um aus dem Kondensator entnommen zu werden, wobei dieses Wasser dann vollkommen sauber ist. Es braucht nur noch mit Mineralien angereichert zu werden, um Trinkwasser zu werden, oder kann auch als destilliertes Wasser, etwa für medizinische Zwecke, verwendet werden.

Nachfolgend wird die vorliegende Erfindung unter Bezugnahme auf die anliegenden Figuren näher erläutert, wobei weitere Vorteile und Merkmale gemäß der vorliegenden Erfindung bzw. Kombinationserfindung ersichtlich werden. Es zeigen:
- Fig. 1: eine bevorzugte Ausführungsform einer erfindungsgemäß ausgestalteten Vorrichtung in einer schematischen Seitenansicht;
- Fig. 2: die Ausführungsform gemäß Fig. 1 mit teilweise ausgezogener Desinfektionslade;
- Fig. 3: eine schematische, perspektivische Darstellung der Verbindungsorgane und deren Ent- bzw. Versorgungsleitungen;
- Fig. 4: eine schematische Darstellung einer Vakuum-Anlage einer erfindungsgemäß ausgestalteten Vorrichtung;
- Fig. 5: die Gegenüberstellung einer Draufsicht und einer Seitenansicht der Ausführungsform gemäß den Fig. 1 und 2; und
- Fig. 6: eine Injektionsnadel gemäß einer Ausführungsform der vorliegenden Erfindung.

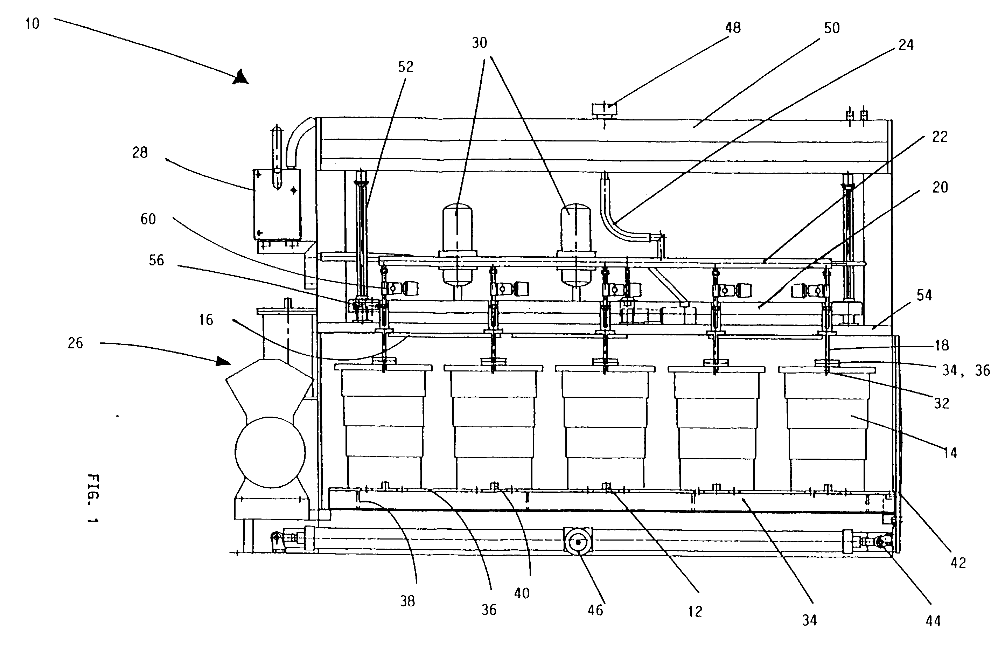

In Fig. 1 ist eine bevorzugte Ausführungsform gemäß der vorliegenden Erfindung, d.h. eine Vorrichtung 10 zur Entsorgung vorzugsweise infektiöser Abfälle, dargestellt. In der Vorrichtung 10 sind Stellplätze 12 vorgesehen, auf denen Behälter 14 abgestellt werden können. Die Behälter 14 enthalten in der Regel Sondermüll. Der Sondermüll kann infektiöse und auch toxische Abfälle, beispielsweise Krankenhausabfälle, etc. enthalten.

Hinter den Platten 16 verbergen sich Energiequellen, die Energie für einen Sterilisationsvorgang zur Verfügung stellen können. Hinter den Platten 16 sind in der Regel Generatoren vorgesehen, die eine relativ weitreichende Energie, die auch durch die Wände der Behälter 14 hindurchgehen kann, zur Verfügung stellen. Als Generatoren empfehlen sich dabei folglich Mikrowellen-Generatoren, gegebenenfalls auch Strahlungsquellen für Röntgen oder -Strahlung, wobei die Strahlung dann intensiv genug sein muß, um Viren, Bakterien oder dergleichen zu zerstören. In der Regel werden jedoch Mikrowellen-Generatoren zu bevorzugen sein.

Über Verbindungsorgane 18 können Fluide in die Behälter 14 eingeführt bzw. aus diesen entsorgt werden. Die Verbindungsorgane 18 weisen eine Schneidfläche 32 auf, die stabil und scharf genug sein muß, um die Deckel bzw. Wände der Behälter 14 punktieren zu können. Um eine dichte Verbindung zwischen den Behältern und den Sterilisationseinrichtungen der Entsorgungsvorrichtung 10 zustande bringen zu können, ist oberhalb der Schneidfläche 32 eine Dichteinrichtung 34, 36 angeordnet. Diese Dichteinrichtung kann beispielsweise eine Silikonscheibe 34 aufweisen, die durch einen Stahlring, vorzugsweise einen V2A-Stahlring 36, abgestützt wird, damit sich die Dichtung 34 beim Anpressen gegen die Wand bzw. den Deckel des Behälters 14 nicht verformen kann. Der Deckel des Abfallbehälters kann mit keilförmigen Vertiefungen mit Rillen oder sonstigen Profilen versehen sein, um eine bessere Abdichtung des Funktionsbereiches zwischen der Silikonscheibe und dem Deckel gegenüber der Umwelt zu ermöglichen.

Die Verbindungsorgane 18 sind über Sammelleitungen bzw. Sammelleitungsabschnitte 20, 22 an verschiedene Ver- bzw. Entsorgungsabschnitte der erfindungsgemäßen Vorrichtung 10 angeschlossen. Über Ventile 60 können die Verbindungsorgane 18 beispielsweise an einen Wassertank 50 bzw. an eine Vakuumpumpe bzw. einen Entsorgungsblock 26 angeschlossen werden. Dabei können die Ventile 60 für die Bereitstellung einer vorgebbaren Wassermenge geöffnet bzw. geschlossen werden, um Wasser aus dem Wassertank 50 über die Versorgungsleitungen 22 und die Verbindungsorgane 18 in die punktierten Behälter 14 einzuleiten. Das Wasser kann vorgewärmt oder aber auch kalt sein. Sobald eine entsprechende Wassermenge in den jeweiligen Behälter 14 eingeleitet worden ist, werden die jeweiligen Ventile 60 geschlossen und die Mikrowellen-Generatoren (nicht dargestellt) treten in Aktion. Über an den Spitzen der Verbindungsorgane 18 oder in einem mit den Behältern 14 in unmittelbarem Fluidkontakt stehenden Abschnitten innerhalb der Verbindungsorgane 18 kann die Temperatur bzw. der Feuchtigkeitsgehalt innerhalb der Behälter 14 gemessen werden. Sollte sich herausstellen, daß die in den Behälter eingeleitete Wassermenge nicht ausreicht, um die für einen Sterilisationsvorgang erforderliche Heißdampf-Atmosphäre zur Verfügung zu stellen, kann ein jeweiliges Ventil 60 geöffnet werden, um eine zusätzliche, vorgebbare Wassermenge aus dem Wassertank 50 in den jeweiligen Behälter 14 einzuleiten. Der Sterilisationsvorgang wird dann für die Behälter 14 oder aber auch für ausgewählte Behälter 14 fortgesetzt, bis vorgeschriebene Parameter gemessen werden, die eine innerhalb eines jeweiligen Behälters 14 eingestellte Sterilisations-Atmosphäre garantieren.

Die Behälter 14 lasen sich vorzugsweise hermetisch abdichten und sind auch unter Druck, etwa bei einem Druck bzw. Überdruck von ca. 0,1 bis 1 Bar bzw. einem Unterdruck noch ausreichend dicht, um die Umwelt nicht zu kontaminieren.

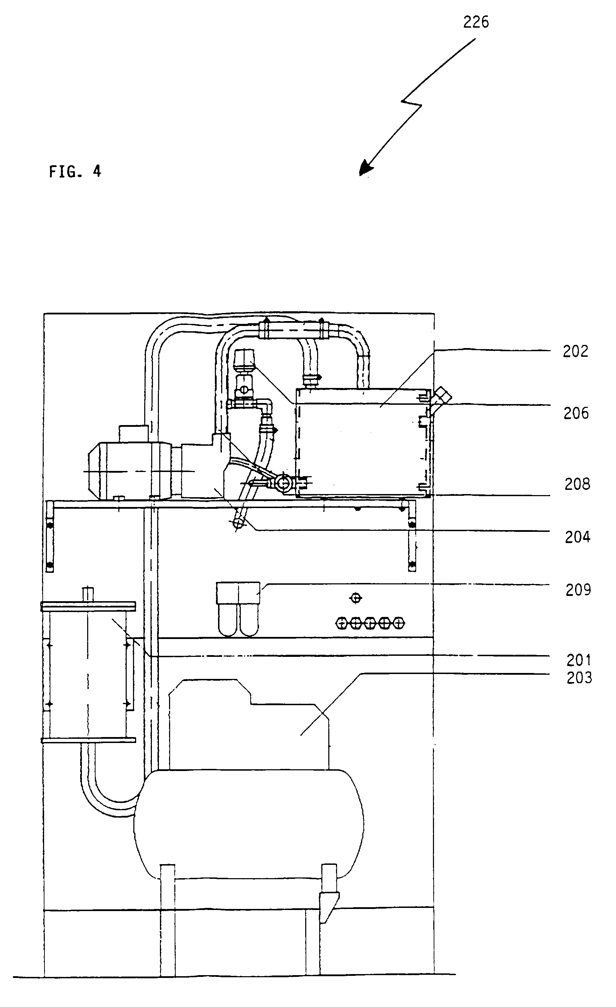

Bevor und gegebenenfalls nachdem der Sterilisationsvorgang vorzugsweise mittels der Wasserdampf-Atmosphäre stattgefunden hat, kann der jeweilige Behälter 14 durch ein Vakuumsystem 26 über Vakuumleitungen 20 und die Verbindungsorgane 18 evakuiert werden. Die dabei abgezogene Atmosphäre kann zunächst durch einen Kondensator, beispielsweise einen Kaltwassertank (siehe Fig. 4) geleitet werden, um Feuchtigkeit und kondensierbare Bestandteile aus der evakuierten Atmosphäre innerhalb der Behälter 14 zu eliminieren. Ist zu befürchten, daß noch aktive bakterielle, virale bzw. toxische Bestandteile in der aus den jeweiligen Behältern 14 abgezogenen Atmosphäre enthalten sind, so kann das vorzugsweise im wesentlichen bzw. vollständig von kondensierbaren Bestandteilen gereinigte Abgas noch durch Fein- bzw. Feinstfilter 30 hindurchgeleitet werden, damit keine unsterilisierten, möglicherweise baktierellen bzw. toxischen Abfallbestandteile mit der aus den Behältern 14 abgepumpten Atmosphäre an die Umwelt gelangen können.

In dem Kondensator 28 gegebenenfalls kondensierte Wasserbestandteile können in den Wasservorratstank 50 zurückgeleitet werden. Am Wasservorratstank 50 ist ein Wasserstandsfühler 48 vorgesehen, der signalisieren kann, wenn einer oder mehrere bestimmte Wasserpegel unterschritten worden sind. Der Wassertank 50 ist über einen flexiblen Anschlußschlauch 24 an die Versorgungsleitung 22 und damit an die Verbindungsorgane 18 angeschlossen.

Die Ver- bzw. Entsorgungsleitungen 20, 22 sind als im wesentlichen starre Hohlrahmenkonstruktionen ausgebildet, die bevorzugt mit den Verbindungsorganen bzw. Injektionsnadeln 18 in Richtung auf die Behälter 14 abwärts bewegt und nach einem erfolgreichen Sterilisationsvorgang wieder aufwärts bewegt werden. Die rahmenartige Ausgestaltung der Ver- bzw. Entsorgungsleitungen 20, 22 vermittelt der erfindungsgemäßen Vorrichtung 10 zusätzliche Stabilität und eine außerordentliche Sicherheit, was ganz besonders bei der Entsorgung stark infektiöser Abfälle ein ganz besonders wesentlicher Vorteil der vorliegenden Erfindung ist. Die die Verbzw. Entsorgungsleitungen 20, 22 aufweisende Konstruktion kann über eine mechanische Mimik 52, 56, die von einem Stützrahmen 54 getragen ist, aufwärts bzw. abwärts bewegt werden. Sensoren bzw. Wegstreckendetektoren können für die Aufwärts- bzw. Abwärtsbewegung über eine vorgegebene Strecke sorgen. Diese erfindungswesentliche Konstruktion, bei der auch vorzugsweise Leitungen 18, 20, 22 mit großen Querschnitten und möglichst mittigen Anschlüssen gewählt werden, ergibt eine hohe Zuverlässigkeit und Dichtigkeit, was einen bei der Entsorgung maßgeblichen Vorteil darstellt. Auch lassen sich hier in den Behältern 14, beispielsweise acht, zehn oder zwölf Behälter 14, zuverlässig identische Sterilisationsparameter einstellen.

Die Behälter 14 sind auf einer schubladenartigen Konstruktion 34 abgestellt, auf der die Stellplätze 12 der Behälter 14 plaziert sind. Die Oberfläche der Sterilisations- bzw. Desinfektionslade 34 weist eine zumindest überwiegend aus dielektrischem Material bestehende Platte 36 auf, die Markierungen in Form von Erhebungen 40 aufweist, die ein Pandant am Bodenabschnitt eines jeweiligen Behälters 14 haben. Auf diese Weise lassen sich die Behälter genau anordnen, so daß die Schneidfläche 32 eines jeweiligen Verbindungsorgans 18 den jeweiligen Behälter 14 in einem sehr eingeschränkten Bereich punktieren kann und die Behälter gegenüber den Mikrowellengeneratoren bzw. den Abstrahlflächen 16 der Mikrowellengeneratoren jeweils genau und definiert zu liegen kommen. Die Platte 36 kann durch zumindest teilweise aus dielektrischem Material, vorzugsweise der gleichen Art wie die Platte 36, bestehenden Abstandshaltern bzw. Füßen angeordnet sein. Da die Stellfläche bzw. Abstellplatte 36 relativ groß ist, kann es durch die räumlich eng begrenzte Wirkung der jeweiligen Mikrowellen-Generatoren, die jeweils einem Behälter 14 zugeordnet sein können, zu großen Temperaturgradienten kommen. Deshalb, damit das Material der Platte 36 durch die starken Wärmegradienten und die damit verbundenen Spannungen nicht zerstört wird, sollte die Platte 36 aus einem hochwertigen Glas, Polycarbonat, Kunststoff bzw. Teflon oder dergleichen zumindest teilweise bestehen. Die Desinfektions- bzw. Sterilisationslade 34 weist an ihrem einen Ende einen Deckel bzw. ein Abschlußstück 42 auf, das den Desinfektionsraum bzw. die Desinfektionskammer, die zumindest Teile der Vorrichtung 10 umschließt, gegenüber der Außenwelt abschirmt, um keine möglicherweise gefährlichen Mikrowellenlecks zuzulassen. Die Desinfektionskammer kann, je nach der gewählten Strahlungsart aus Edelstahl, Blei oder dergleichen sein.

Die Sterilisiationslade 34 kann beispielsweise über eine Hydraulik 46, 44 aus der Sterilisationskammer heraus- und in diese wieder hineingefahren werden. Über das zusätzliche Gelenk 44 kann der Deckel 42 mit einer zusätzlichen Kraft an den angrenzenden Bereich der Sterilisationskammer angepreßt werden. Außerdem kann der Deckel 42 über das Gelenk 44 als Rampe verwendet werden.

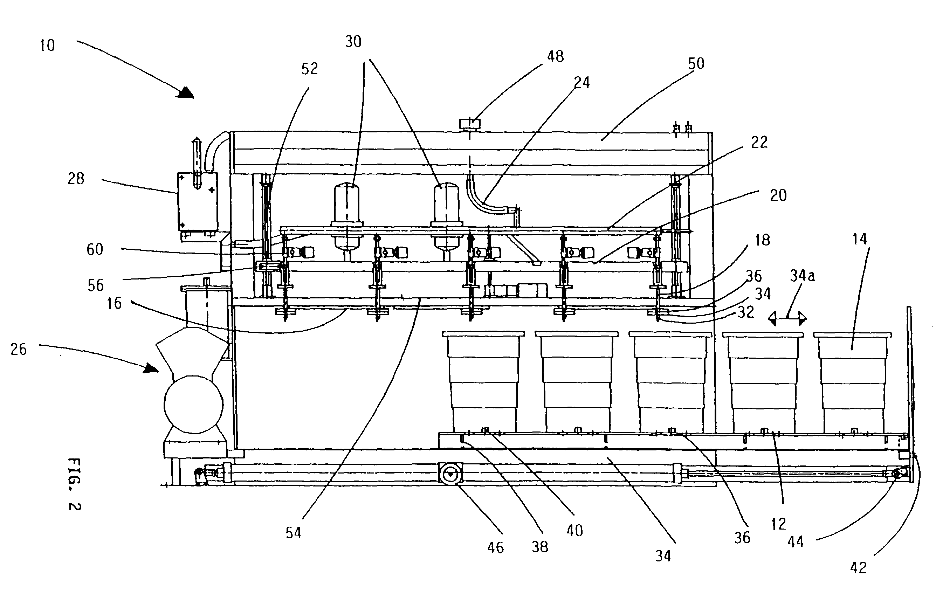

In Fig. 2, wie auch in allen anderen Figuren, bezeichnen gleiche Bezugszeichen gleiche oder zumindest funktionsgleiche Teile. Gegebenenfalls können auch um jeweils 100 heraufgesetzte Bezugszeichen gleiche bzw. zumindest funktionsgleiche Teile bezeichnen (beispielsweise 14 und 114).

In Fig. 2 ist die Vorrichtung 10 gemäß Fig. 1 mit einer teilweise ausgefahrenen Sterilisationslade 34 dargestellt, wobei der Doppelpfeil 34a die möglichen Bewegungsrichtungen der Sterilisationslade 34 andeutet. Die Verbindungsorgane 18 sind bei dieser Darstellung in die Behälter 14 noch nicht hineingestoßen worden bzw. aus diesen bereits wieder herausgezogen worden. Im ersten Falle enthalten die Behälter 14 noch unsterilisierte Abfälle, während sie im zweiten Fall sterilisierte Abfälle enthalten, die als Hausmüll weiterbehandelt werden können.

Wie zu erkennen ist, ist der die Versorgungs- und Entsorgungsleitungen 20, 22 enthaltende Rahmen gegenüber dem Tragrahmen 54 abgestützt mit den Verbindungsorganen 18 zusammen in einer hochgefahrenen Stellung, in der die Behälter zusammen mit der Sterilisationslade 34 frei unter den Schneidflächen 32 entlang des Doppelpfeils 34a bewegbar sind.

In Fig. 2 ist die als Funktionsrahmen 100 ausgebildete gemeinschaftlich bewegbare Einheit aus Ver- bzw. Entsorgungsleitungen 120, 122 mit den daran vorgesehenen Verbindungsorganen 118 dargestellt. Der Versorgungsrahmen 122, über den Wasser aus dem Wassertank 15 zu den Verbindungsorganen 118 geleitet werden kann, läßt sich über die Ventile 160 an die Verbindungsorgane 118 anschließen bzw. von diesen abkoppeln. Die Entsorgungsleitungen bzw. der den Entsorgungsleitungen zugeordnete Entsorgungsrahmen 120 kann über Filtereinrichtungen 130, beispielsweise Fein- oder Feinstfilter, an einen Pump- bzw. Vakuumblock 26 (siehe Fig. 1 und 2) angeschlossen sein.

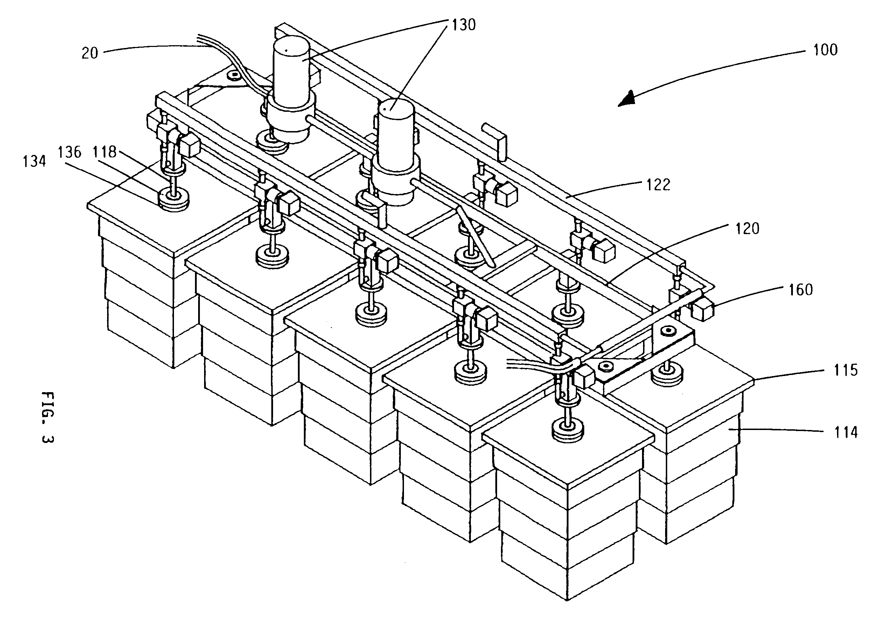

In dem in Fig. 3 dargestellten Betriebsmoment sind die Verbindungsorgane 118 heruntergefahren, d.h. die Schneidflächen 32 (siehe Fig. 1 und 2) sind durch Deckel 115 der Behälter 114 hindurchgestoßen worden, so daß das Desinfektionssystem der erfindungsgemäß ausgestalteten Vorrichtung 10 mit den Behältern 114 in Verbindung steht. Die Silikon-Dichtscheibe 134 bzw. die Edelstahlscheibe 136 sorgen für die erforderliche dichte Anbindung des Sterilisationssystems an die jeweiligen Behälter 114.

In der Fig. 4 ist das Pump- bzw. Vakuumsystem 226 der bevorzugten Ausführungsform gemäß der vorliegenden Erfindung wiedergegeben. Ein Kaltwassertank 202 kann als Kondensator für in der aus den Behältern 14, 114 abgesaugten Atmosphäre dienen. Gegebenenfalls kann hier auch ein Kühlaggregat vorgesehen sein. Ventile 206, 208 dienen zur jeweiligen Anbindung von Rohrleitungen aneinander bzw. zur Entlüftung, Notentlüftung bzw. zur Versorgung mit frischem Kühlmittel bzw. Wasser.

Eine Vakuumpumpe 204, die zumindest ein Vakuum im Vorvakuumbereich erzeugen kann, saugt die aus den Behältern 14, 114 stammende Atmosphäre durch den Kühlwasserbehälter 202, beispielsweise über eine schlangenförmig durch den Kühlbehälter 202 hindurchgelegte Leitung hindurch. Das Kondensat kann aus dem Kondensator bzw. Kühlmittelbehälter 202 beispielsweise über die Ventileinrichtung 208 abgezogen werden.

Ein Kompressor 203 versorgt die jeweils betroffenen Systeme der erfindungsgemäß ausgestalteten Vorrichtung mit Druckluft. Die letztlich aus den Behältern abgezogene Atmosphäre kann noch durch einen Geruchsfilter 201, beispielsweise einem Aktivkohlefilter oder dergleichen, hindurchgedrückt werden, um eine Geruchsbelästigung der Umwelt zu vermeiden.

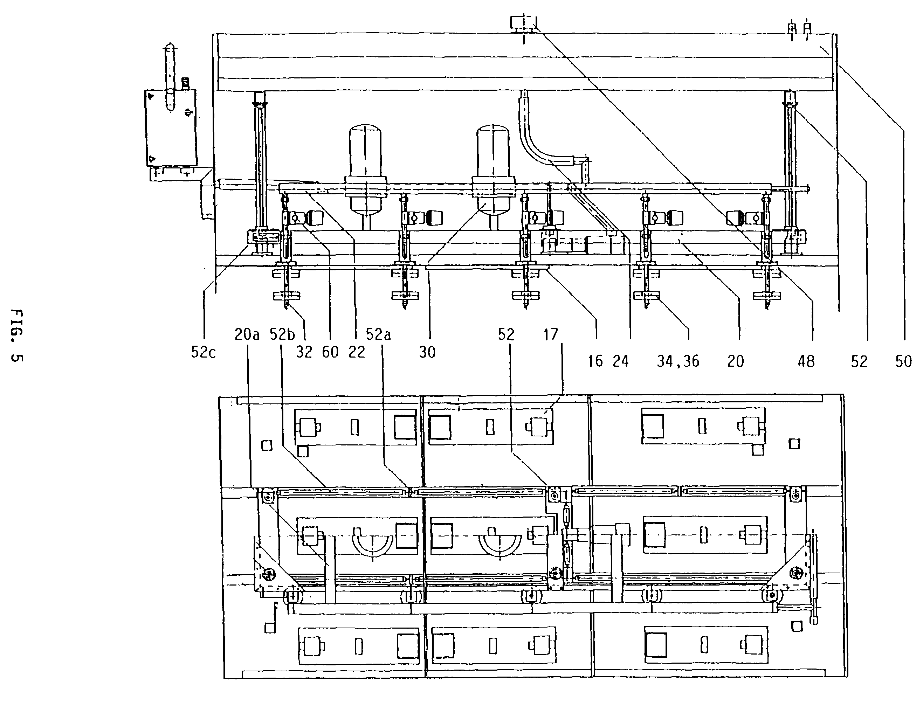

In Fig. 5 ist die Ausführungsform gemäß den Fig. 1 und 2 nochmals dargestellt. Zusätzlich zu den in den Fig. 1 und 2 wiederzufindenden Merkmalen ist in der Fig. 5 ein Hohlraum 17 für die Mikrowellengeneratoren dargestellt. In Abhängigkeit zu der Geometrie der Behandlungskammer der erfindungsgemäßen Vorrichtung sind in der hier wiedergegebenen Anordnung gegenüber insgesamt zehn Behältern 14, 114 lediglich neun Mikrowellengeneratoren angeordnet, die in der Behandlungskammer ein ausreichendes Feld erzeugen, um die nötige Dampfatmosphäre, die für die Sterilisation der Behälterinhalte erforderlich ist, bereitzustellen.

In Fig. 5 sind ferner verschiedene Antriebswellen bzw. Gelenke 52a, b, c zu erkennen, die für die synchrone Bewegung des besonders gut aus Fig. 3 ersichtlichen Funktionsrahmens 100 erforderlich sind. Für die Bewegung sind sowohl Hydrauliken als auch mechanische Spindelantriebe und vergleichbare Vorrichtungen verwendbar.

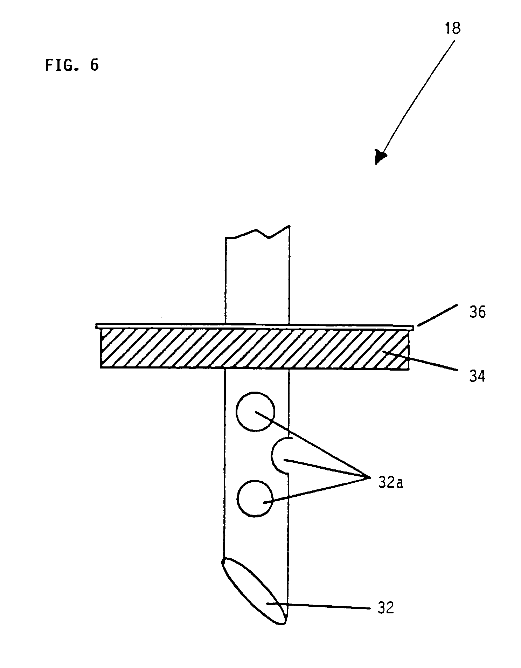

In Fig. 6 ist eine Injektionsnadel 18 erkennbar, die gemäß der vorliegenden Erfindung ausgestaltet ist. Diese weist eine Schneidfläche 32 und einen unteren Wasseraustritt auf. Da dieser beim Einstechen in einen Behälter 14 verstopfen kann, wenn er sich etwa mit Abfall zusetzt, sind an den Seitenwänden seitliche Wasseraustritte bzw. seitliche Pumpöffnungen 32a vorgesehen, die die Einstellung der erforderlichen Desinfektionsbedingungen auch dann erlauben, wenn die Schneidfläche 32 mit dem unteren Wasseraustritt zugesetzt ist. Am unteren Wasseraustritt der Injektionsnadel 18 können auch noch Meßfühler angeordnet sein, beispielsweise, um die Temperatur, den Druck oder die Feuchtigkeit und gegebenenfalls weitere Parameter innerhalb der Behälter 14 während des Desinfektionsvorganges zu messen.

Oberhalb der seitlichen Austrittsöffnungen 32 a und der unteren Austrittsöffnung 32 ist eine Dichtung 34, 36, vorzugsweise ein Silikon-Dichtring 34, angeordnet, der mit einem Stahlring 36, insbesondere einem V2A-Ring, abgestützt werden kann.

## Patentansprüche

1. Vorrichtung (10) für die Entsorgung von vorzugsweise infektiösen Abfällen,
mit einem Behandlungsabschnitt mit Stellplätzen (12) für die Abfälle aufnehmende Behälter (14),
mit mindestens einer Energie- bzw. Wärmequelle (16), und
mit mindestens einem Verbindungsorgan (18), vorzugsweise einer Einstechhohl- bzw. Injektionsnadel, die an mindestens eine Ver- bzw. Entsorgungsleitung (20, 22, 24) anschließbar ist,
**gekennzeichnet** durch die folgenden Merkmale:
der Behandlungsabschnitt weist vorzugsweise für jede Versorgungsleitung bzw. Entsorgungsleitung mindestens einen Sammelleitungsabschnitt (20, 22, 24) auf.

2. Vorrichtung nach Anspruch 1, dadurch **gekennzeichnet,** daß die vorzugsweise sämtlichen Sammelleitungsabschnitte (20, 22, 24) zusammen mit den Verbindungsorganen (18) zumindest vertikal bewegbar sind.

3. Vorrichtung nach Anspruch 1 oder 2, dadurch **gekennzeichnet,** daß der bzw. die Sammelleitungsabschnitte eine rahmenartige Anordnung bilden.

4. Vorrichtung nach einem der Ansprüche 1 bis 3, dadurch **gekennzeichnet,** daß die Sammelleitungsabschnitte (20, 22, 24), die flüssige und/oder gasförmige Fluide zu- bzw. ableiten, im wesentlichen mittig an die jeweilige Ver- bzw. Entsorgung (25, 28, 30) anschließbar sind, so daß die die zu ver- bzw. entsorgenden Fluide zu den Behältern (14) vorzugsweise im wesentlichen gleiche Strecken zurückzulegen haben.

5. Vorrichtung nach einem der Ansprüche 1 bis 4, dadurch **gekennzeichnet,** daß die jeweiligen Sammelleitungsabschnitte in einem jeweiligen oder gemeinsamen Sammelrohr bzw. einer Sammeleinrichtung (20, 22) angeordnet sind, an der vorzugsweise auch die Verbindungsorgane (18) abgestützt sind.

6. Vorrichtung nach einem der Ansprüche 1 bis 5, dadurch **gekennzeichnet,** daß die Verbindungsorgane (18) ein Verbindungsende, insbesondere eine Einstichnadel bzw. Kanüle (32), aufweisen, an dem mehrere Leitungen enden bzw. münden.

7. Vorrichtung nach einem der vorstehenden Ansprüche, dadurch **gekennzeichnet,** daß am Verbindungsende eine Schneidfläche angeordnet ist und/oder die Seitenwände der Verbindungsorgane (18, 32) die Anschlußöffnungen der Leitungen (20, 22, 24) aufweisen.

8. Vorrichtung nach einem der vorstehenden Ansprüche, dadurch **gekennzeichnet,** daß oberhalb des Verbindungsendes und/oder der Anschlußöffnungen eine Dichteinrichtung (34, 36), beispielsweise eine Silikonscheibe (34), ein Silikonkissen, eine Gummiplatte oder dergleichen, angeordnet ist, die einen Behälter (14) im eingestochenen Zustand der Verbindungsorgane (18, 32) abdichten kann.

9. Vorrichtung nach einem der vorstehenden Ansprüche, dadurch **gekennzeichnet,** daß der bzw. die Sammelleitungsabschnitte (20, 22) längs oder senkrecht zu der Formationsordnung der Behälter (14) erstreckbar sind.

10. Vorrichtung nach einem der Ansprüche 1 bis 9, dadurch **gekennzeichnet,** daß die Behälter (14) und/oder die Verbindungsorgane (18, 32) vertikal bewegbar sind.

11. Vorrichtung nach einem der Ansprüche 1 bis 10, dadurch **gekennzeichnet,** daß Fluid bzw. flüssige und/oder gasförmige Medien über die Leitungen (18, 20, 22, 24) zu- bzw. abführbar sind, insbesondere mindestens jeweils eine Vakuumleitung, eine Wasserzuleitung, eine Druckluftzuleitung, eine Dampfableitung und/oder elektrische Versorgungs- und Steuerleitungen angeordnet sind.

12. Vorrichtung nach einem der vorstehenden Ansprüche, dadurch **gekennzeichnet,** daß die Ver- bzw. Entsorgungsleitungen (20, 22) einen großen Querschnitt aufweisen.

13. Vorrichtung nach einem der vorstehenden Ansprüche, dadurch **gekennzeichnet,** daß an den Verbindungsorganen (18, 32) Meßfühler, vorzugsweise an deren einstechbaren Spitzen (32), angeordnet sind, vorzugsweise für eine Temperatur-, eine Druck- und/oder eine Feuchtemessung.

14. Vorrichtung nach einem der vorstehenden Ansprüche, dadurch **gekennzeichnet,** daß in der Entsorgungsleitung für den Wasserdampf bzw. in der Vakuumleitung eine Filtereinrichtung, z.B. ein Feinstfilter, angeordnet ist, vorzugsweise, um Bakterien, Viren und dergleichen zurückzuhalten.

15. Vorrichtung nach Anspruch 14, dadurch **gekennzeichnet,** daß vor der Filtereinrichtung eine Kühleinrichtung, vorzugsweise ein Kondensator angeordnet ist.

16. Vorrichtung nach einem der vorstehenden Ansprüche, dadurch **gekennzeichnet,** daß die kondensierte Flüssigkeit sterilisierbar und wieder verwendbar behandelbar ist.

17. Vorrichtung nach einem der vorstehenden Ansprüche, dadurch **gekennzeichnet,** daß die den Abfall aufnehmenden Behälter über die Verbindungsorgane und die Wasserleitungen und/oder die Vakuumleitungen in Verbindung bringbar sind, damit ein Ausgleich zwischen den Behältern mit infektiösem Abfall stattfinden kann, um während der Desinfektionsbehandlung vergleichmäßigte Behandlungsbedingungen einstellen zu können.

18. Vorrichtung nach einem der vorstehenden Ansprüche, dadurch **gekennzeichnet,** daß die Stellplätze (12) für die die Abfälle aufnehmenden Behälter auf einer bewegbaren Einrichtung (34) angeordnet sind, die vorzugsweise schubladenartig in die Behandlungskammer einführbar ist.

19. Vorrichtung nach Anspruch 18, dadurch **gekennzeichnet,** daß die Einrichtung (34) eine Auflageplatte (36) aufweist und zumindest diese ein im wesentlichen dielektrisches Material, vorzugsweise Glas, Polycarbonat, Teflon und/oder dergleichen, aufweist bzw. daraus besteht.

20. Vorrichtung nach einem der vorstehenden Ansprüche, dadurch **gekennzeichnet,** daß die Platte auf mindestens einem Abstandshalter (38) abgestützt ist und vorzugsweise über diesen aus der Behandlungskammer bzw. in die Behandlungskammer bewegbar ist, wobei der Abstandshalter zumindest teil- bzw. abschnittsweise aus dem gleichen Material ist, wie die Platte.

21. Vorrichtung nach einem der vorstehenden Ansprüche, dadurch **gekennzeichnet,** daß auf der Platte Vertiefungen und/oder Erhebungen (40) ausgebildet sind, die vorzugsweise eine Positionierung der die Abfälle aufnehmenden Behälter zu den Verbindungsorganen und deren Abstützung ermöglichen.

22. Vorrichtung nach einem der vorstehenden Ansprüche, dadurch **gekennzeichnet,** daß an einem Endbereich der bewegbaren Einrichtung (34) bzw. der Platte ein Endabschnitt (42) angeordnet ist, der den Behandlungsbehälter im wesentlichen verschließt, wenn die bewegbare Einrichtung (34) in dem Behälter in einer Behandlungsstellung ist.

23. Vorrichtung nach einem der vorstehenden Ansprüche, dadurch **gekennzeichnet,** daß in der Behandlungskammer vorzugsweise für jeden die Abfälle aufnehmenden Behälter (14) ein Mikrowellen-Generator (16) anordenbar ist.

24. Vorrichtung nach einem der vorstehenden Ansprüche, dadurch **gekennzeichnet,** daß der Endabschnitt (42) zu den angrenzenden Bereichen der Behandlungskammer dicht, insbesondere mikrowellendicht, in Anlage bringbar ist.

25. Vorrichtung nach einem der vorstehenden Ansprüche, dadurch **gekennzeichnet,** daß der Endabschnitt (42) eine Vorspannung aufweist, der den Endabschnitt mit einer verbesserten Dichtwirkung versieht.

26. Vorrichtung nach einem der vorstehenden Ansprüche, dadurch **gekennzeichnet,** daß zumindest einige der Ver- bzw. Entsorgungsleitungen (20, 22) zumindest bereichsweise mit Heizeinrichtungen versehen sind, beispielsweise die Wasserzu- und Kondensatableitungen, mit elektrischen Widerstandsheizungen umwickelt sind.

27. Fahrzeug, dadurch **gekennzeichnet,** daß auf bzw. an diesem die Vorrichtung nach einem der Ansprüche 1 bis 26 angeordnet ist.

28. Fahrzeug nach Anspruch 27, dadurch **gekennzeichnet,** daß im Kopfraum der Behandlungskammer bzw. oberhalb der Behandlungskammer im Fahrzeug ein Wasservorratstank (50) angeordnet ist, der insbesondere mindestens einen Vertiefungsbereich aufweist, der an die Verbindungsorgane (18, 32) anschließbar ist.

## Claims

1. An apparatus (10) for the disposal of preferably infectious waste,
having a treatment portion with designated places (12) for the containers (14) receiving the waste,
having at least one energy or heat source (16), and
having at least one connecting member (18), preferably an insertable cannula or injection needle, which can be connected to at least one supply or disposal pipe (20, 22, 24),
**characterised by** the following features:
the treatment section preferably comprises at least one collecting pipe portion (20, 22, 24) for each supply pipe or disposal pipe.

2. An apparatus according to Claim 1,
**characterised in that** preferably all collecting pipe portions (20, 22, 24) together with the connection member (18) can be moved at least vertically.

3. An apparatus according to Claim 1 or 2,
**characterised in that** the collecting pipe portion or portions form a frame-shaped arrangement.

4. An apparatus according to one of Claims 1 to 3,
**characterised in that** the collecting pipe portions (20, 22, 24), which supply and take away liquid and/or gaseous fluids, can be joined substantially centrally to the respective supply and disposal pipe (26, 28, 40), so that the liquids to be supplied to and removed from the containers (14) have to cover preferably equal distances.

5. An apparatus according to one of Claims 1 to 4,
**characterised in that** the respective collecting pipe portions are disposed in a respective or common collecting pipe or a collection device (20, 22), on which the connecting members (18) are also preferably supported.

6. An apparatus according to one of Claims 1 to 5,
**characterised in that** the connection members (18) comprise a connection end, in particular an insertable needle or cannula (32), at which several pipes end or open.

7. An apparatus according to one of the preceding Claims,
**characterised in that** a cutting face is provided at the connection end and/or the side walls of the connection members (18, 32) comprise the connection openings of the pipes (20, 22, 24).

8. An apparatus according to one of the preceding Claims,
**characterised in that** provided above the connection end and/or the connection openings is a sealing device (34, 36), for example a silicon disc (34), a silicon cushion, a rubber plate or the like, which can seal a container (14) in the inserted state of the connection members (18, 32).

9. An apparatus according to one of the preceding Claims,
**characterised in that** the collecting pipe portion or portions (20, 22) can be extended along or perpendicularly to the arrangement of the containers (14).

10. An apparatus according to one of Claims 1 to 9,
**characterised in that** the containers (14) and/or the connection members (18, 32) can move vertically.

11. An apparatus according to one of Claims 1 to 10,
**characterised in that** fluid or liquid and/or gaseous media can be supplied or carried away via the pipes (18, 20, 22, 24), in particular at least respectively a vacuum pipe, a water pipe, a compressed air pipe, a steam pipe and/or electrical supply and control pipes.

12. An apparatus according to one of the preceding Claims,
**characterised in that** the supply and disposal pipes (20, 22) have a large cross section.

13. An apparatus according to one of the preceding Claims,
**characterised in that** disposed on the connection members (18, 32), preferably on their insertable points, are probes, preferably for a measurement of the temperature, pressure and/or humidity.

14. An apparatus according to one of the preceding Claims,
**characterised in that** a filter device, e.g. a superfine filter, is disposed in the disposal pipe for the steam or in the vacuum pipe, preferably to hold back bacteria, viruses and the like.

15. An apparatus according to Claim 14,
**characterised in that** a cooling appliance, preferably a condenser, is disposed in front of the filter device.

16. An apparatus according to one of the preceding Claims,
**characterised in that** the condensed liquid can be sterilised and can be treated for reuse.

17. An apparatus according to one of the preceding Claims,
**characterised in that** the containers receiving the waste can be brought into communication via the connecting members and the water pipes and/or the vacuum pipes, so that an equalisation can occur between the containers with infectious waste, so as to be able to set equalised treatment conditions during the disinfection treatment.

18. An apparatus according to one of the preceding Claims,
**characterised in that** the designated places (12) for the containers receiving the waste are disposed on a movable appliance (34), which can preferably be introduced in the manner of a drawer into the treatment chambers.

19. An apparatus according to Claim 18,
**characterised in that** the appliance (34) comprises a base plate (36) and at least said plate comprises or is made from a substantially dielectric material, preferably glass, polycarbonate, Teflon and/or the like.

20. An apparatus according to one of the preceding Claims,
**characterised in that** the plate is supported on at least one spacer (38) and can preferably be moved over it out of the treatment chamber or into the treatment chamber, the spacer being made at least partially or in sections from the same material as the plate.

21. An apparatus according to one of the preceding Claims,
**characterised in that** recesses and/or raised portions (40) are constructed on the plate, which preferably enable a positioning of the containers receiving the waste in relation to the connecting members and their supports.

22. An apparatus according to one of the preceding Claims,
**characterised in that** disposed on one end region of the movable appliance (34) or plate is an end portion (42), which substantially closes the treatment container when the movable appliance (34) is in the container in a treatment position.

23. An apparatus according to one of the preceding Claims,
**characterised in the** a microwave generator (16) can be disposed in the treatment chamber, preferably for each container (14) receiving the waste.

24. An apparatus according to one of the preceding Claims,
**characterised in that** the end portion (42) can be brought to tightly abut the adjacent regions of the treatment chamber, in particular in a manner impervious to microwaves.

25. An apparatus according to one of the preceding Claims,
**characterised in that** the end portion (24) comprises a prestressing which provides the end portion with an improved sealing action.

26. An apparatus according to one of the preceding Claims,
**characterised in that** at least some of the supply and disposal pipes (20, 22) are provided at least in some regions with heating appliances, for example the water supply pipes and condensate delivery pipes are wound with electric resistance heating devices.

27. A vehicle,
**characterised in that** the apparatus according to one of Claims 1 to 26 is disposed thereon.

28. A vehicle according to Claim 27,
**characterised in that** a water supply tank, which in particular comprises at least one recessed region which can be connected to the connecting members (18, 32), is disposed in the headroom of the treatment chamber or above the treatment chamber in the vehicle.

## Revendications

1. Dispositif (10) pour l'élimination de déchets de préférence infectieux,
avec une section de traitement comportant des emplacements (12) pour des récipients (14) contenant des déchets,
avec au moins une source d'énergie ou, respectivement, de chaleur (16),
et
avec au moins un organe de liaison (18), de préférence une aiguille creuse de perçage et, respectivement, d'injection, qui peut être relié à au moins une conduite d'amenée et, respectivement, d'évacuation (20, 22, 24),
caractérisé par les caractéristiques suivantes :
la section de traitement comporte de préférence au moins une portion de conduite collectrice (20, 22, 24) pour chaque conduite d'amenée et, respectivement, chaque conduite d'évacuation.

2. Dispositif selon la revendication 1, caractérisé en ce que de préférence toutes les portions de conduites collectrices (20, 22, 24) peuvent être déplacées au moins verticalement conjointement avec les organes de liaison (18).

3. Dispositif selon la revendication 1 ou 2, caractérisé en ce que la ou, respectivement, les portions de conduites collectrices forment un agencement en forme de cadre.

4. Dispositif selon l'une des revendications 1 à 3, caractérisé en ce que les portions de conduites collectrices (20, 22, 24) qui amènent et, respectivement, évacuent les fluides liquides et/ou gazeux peuvent être reliées aux moyens respectifs d'amenée et, respectivement, d'élimination (26, 28, 30) sensiblement en leur milieu, de sorte que les fluides à amener aux récipients (14) et, respectivement, à en évacuer doivent de préférence parcourir des trajets sensiblement identiques.

5. Dispositif selon l'une des revendications 1 à 4, caractérisé en ce que les portions de conduites collectrices sont disposées chacune dans un tube collecteur individuel ou commun ou dans un dispositif collecteur (20, 22) sur lequel, de préférence, les organes de liaison (18) sont également implantés.

6. Dispositif selon l'une des revendications 1 à 5, caractérisé en ce que les organes de liaison (18) comportent une extrémité de liaison, en particulier une aiguille de perçage ou une canule (32) dans laquelle plusieurs conduites aboutissent et, respectivement, débouchent.

7. Dispositif selon une des revendications précédentes, caractérisé en ce qu'à l'extrémité de liaison est disposée une surface coupante et/ou les parois latérales des organes de liaison (18, 32) sont pourvues des orifices de liaison des conduites (20, 22, 24).

8. Dispositif selon une des revendications précédentes, caractérisé en ce qu'au-dessus de l'extrémité de liaison et/ou des orifices de liaison est disposé un dispositif d'étanchéité (34, 36), par exemple une rondelle en silicone (34), un coussin en silicone, une plaque en caoutchouc ou analogue, qui est apte à rendre étanche un récipient (14) une fois que les organes de liaison (18, 32) l'ont transpercé.

9. Dispositif selon une des revendications précédentes, caractérisé en ce que la ou, respectivement, les portions de conduites collectrices (20, 22) peuvent s'étendre dans le sens longitudinal ou transversal par rapport à l'ordre de formation des récipients (14).

10. Dispositif selon l'une des revendications 1 à 9, caractérisé en ce que les récipients (14) et/ou les organes de liaison (18, 32) peuvent être déplacés verticalement.

11. Dispositif selon l'une des revendications 1 à 10, caractérisé en ce que le fluide ou, respectivement, les agents fluides et/ou gazeux peuvent être amenés et/ou évacués par les conduites (18, 20, 22, 24), en particulier au moins par une conduite sous vide, une conduite d'arrivée d'eau, une conduite d'arrivée d'air comprimé, une conduite de sortie de vapeur et/ou des lignes électriques d'alimentation et de commande.

12. Dispositif selon une des revendications précédentes, caractérisé en ce que les conduites d'amenée et, respectivement, d'évacuation (20, 22) présentent une grande section transversale.

13. Dispositif selon une des revendications précédentes, caractérisé en ce que des capteurs de mesure, de préférence pour mesurer la température, la pression et/ou l'humidité, sont implantés sur les organes de liaison (18, 32), de préférence au niveau de leurs pointes de perçage (32).

14. Dispositif selon une des revendications précédentes, caractérisé en ce qu'un dispositif de filtration, par exemple un ultrafiltre, est implanté sur la conduite d'évacuation de la vapeur d'eau et, respectivement, sur la conduite sous vide, de préférence pour retenir les bactéries, les virus et analogues.

15. Dispositif selon la revendication 14, caractérisé en ce qu'un dispositif de refroidissement, de préférence un condenseur, est disposé en amont du dispositif de filtration.

16. Dispositif selon une des revendications précédentes, caractérisé en ce que le liquide condensé peut être traité pour pouvoir être stérilisé et réemployé.

17. Dispositif selon une des revendications précédentes, caractérisé en ce que les récipients contenant les déchets peuvent être mis en communication par l'intermédiaire des organes de liaison et des conduites d'eau et/ou des conduites sous vide pour qu'un équilibre entre les récipients de déchets infectieux puisse s'instaurer afin de pouvoir créer des conditions de traitement uniformisées lors du traitement de désinfection.

18. Dispositif selon une des revendications précédentes, caractérisé en ce que les emplacements (12) destinés aux récipients contenant les déchets sont disposés sur un dispositif mobile (34) qui peut rentrer dans la chambre de traitement de préférence à la manière d'un tiroir.

19. Dispositif selon la revendication 18, caractérisé en ce que le dispositif (34) comporte une plaque porteuse (36) et au moins celle-ci comprend ou, respectivement, est réalisée dans une matière sensiblement diélectrique, de préférence du verre, du polycarbonate, du Téflon et/ou analogue.

20. Dispositif selon une des revendications précédentes, caractérisé en ce que la plaque prend appui sur au moins une entretoise (38) et peut, de préférence par l'intermédiaire de celle-ci, sortir hors de la chambre de traitement et, respectivement, y entrer, l'entretoise étant réalisée en partie ou, respectivement, par portions dans la même matière que la plaque.

21. Dispositif selon une des revendications précédentes, caractérisé en ce que sur la plaque sont ménagées des parties en creux et/ou en relief (40) qui permettent de positionner les récipients contenant les déchets par rapport aux organes de liaison et de les maintenir.

22. Dispositif selon une des revendications précédentes, caractérisé en ce que dans une zone extrême du dispositif mobile (34) et, respectivement, de la plaque est disposée une portion extrême (42) qui ferme sensiblement l'enceinte de traitement lorsque le dispositif mobile (34) est en position de traitement dans l'enceinte.

23. Dispositif selon une des revendications précédentes, caractérisé en ce qu'un générateur de micro-ondes (16) peut être prévu dans la chambre de traitement de préférence pour chacun des récipients (14) contenant les déchets.

24. Dispositif selon une des revendications précédentes, caractérisé en ce que la portion extrême (42) peut être appliquée contre les zones adjacentes de la chambre de traitement de manière étanche, en particulier étanche aux micro-ondes.

25. Dispositif selon une des revendications précédentes, caractérisé en ce que la portion extrême (42) présente une précontrainte qui lui confère une meilleure étanchéité.

26. Dispositif selon une des revendications précédentes, caractérisé en ce qu'au moins certaines des conduites d'amenée et, respectivement, d'évacuation (20, 22) sont pourvues, au moins par endroits, de dispositifs de chauffage, par exemple les conduites d'arrivée d'eau et d'évacuation du condensat sont enveloppées de chauffages par résistances électriques.

27. Véhicule, caractérisé en ce que le dispositif selon l'une des revendications 1 à 26 est disposé sur ou dans celui-ci.

28. Véhicule selon la revendication 27, caractérisé en ce qu'un réservoir d'eau (50) comportant en particulier au moins une zone en creux apte à être reliée aux organes de liaison (18, 32) est disposé dans l'espace supérieur de la chambre de traitement ou, respectivement, au-dessus de la chambre de traitement dans le véhicule.
